# EUROPÄISCHE PATENTSCHRIFT

(11) **EP 2 766 472 B1**
(45) Veröffentlichungstag und Bekanntmachung des Hinweises auf die Patenterteilung: **15.03.2017**
(21) Anmeldenummer: 12772328.6
(22) Anmeldetag: 12.10.2012
(51) Int. Cl.: C12N 1/20, C12R 1/23, C12R 1/225, A61K 8/99, A61K 35/74, A61K 35/747, A61Q 11/00

(54) **NEUE MILCHSÄUREBAKTERIEN UND DIESE ENTHALTENDE ZUSAMMENSETZUNGEN**
NOVEL LACTIC ACID BACTERIA AND COMPOSITIONS CONTAINING SAME
NOUVEAUX LACTOBACILLES ET COMPOSITIONS LES CONTENANT

(30) Priorität: 14.10.2011 DE 102011116325
(43) Veröffentlichungstag der Anmeldung: 20.08.2014
(73) Patentinhaber: Orochemie GmbH & Co. KG, 70806 Kornwestheim (DE)
(72) Erfinder: ZIMMERMANN, Frank, 73733 Esslingen (DE); SCHNEIDER, Axel, 71711 Steinheim (DE); NAPHOLZ, Christa, 71642 Ludwigsburg (DE); LUCHTERHANDT, Thomas, 88696 Owingen (DE); SPAETH, Kathrin, 70184 Stuttgart (DE); ACHELWILM, Christiane, 49584 Fürstenau (DE); BOLOTINA, Natalia, 10777 Berlin (DE); HOLZ, Caterina, 10405 Berlin (DE); LANG, Christine, 14057 Berlin (DE)
(74) Vertreter: Witte, Weller & Partner Patentanwälte mbB
(86) Internationale Anmeldenummer: PCT/EP2012/070301
(87) Internationale Veröffentlichungsnummer: WO 2013/053907

(56) Entgegenhaltungen:
- EP-A1- 2 133 414
- WO-A1-2005/120527
- WO-A1-2006/022471
- JP-A- 2010 053 062
- JP-A- 2010 124 772
- US-A1- 2003 077 814
- TEUGHELS WIM ET AL: "Probiotics and oral healthcare", PERIODONTOLOGY 2000, MUNKSGAARD INTERNATIONAL PUBLISHERS, DK, Bd. 48, 7. August 2008 (2008-08-07), Seiten 111-147, XP002594143, ISSN: 0906-6713
- BONIFAIT LAETITIA ET AL: "Probiotics for oral health: myth or reality?", JOURNAL (CANADIAN DENTAL ASSOCIATION) OCT 2009, Bd. 75, Nr. 8, Oktober 2009 (2009-10), Seiten 585-590, XP002688911, ISSN: 1488-2159
- KODUGANTI REKHA RANI ET AL: "Probiotics and prebiotics in periodontal therapy.", INDIAN JOURNAL OF DENTAL RESEARCH : OFFICIAL PUBLICATION OF INDIAN SOCIETY FOR DENTAL RESEARCH 2011 MAR-APR, Bd. 22, Nr. 2, März 2011 (2011-03), Seiten 324-330, XP008158675, ISSN: 1998-3603
- TEUGHELS W ET AL: "Bacteria interfere with A. actinomycetemcomitans colonization", JOURNAL OF DENTAL RESEARCH, Bd. 86, Nr. 7, Juli 2007 (2007-07), Seiten 611-617, XP008158684, ISSN: 0022-0345
- DATABASE WPI Week 201021 Thomson Scientific, London, GB; AN 2010-C59155 XP002688914, & JP 2010 053062 A (SUNSTAR CHEM IND CO LTD) 11. März 2010 (2010-03-11)
- R. TEANPAISAN ET AL: "Inhibitory effect of oral Lactobacillus against oral pathogens", LETTERS IN APPLIED MICROBIOLOGY, Bd. 53, Nr. 4, 1. Oktober 2011 (2011-10-01), Seiten 452-459, XP055047020, ISSN: 0266-8254, DOI: 10.1111/j.1472-765X.2011.03132.x
- MI-SUN KANG ET AL: "Inhibitory effect of Lactobacillus reuteri on periodontopathic and cariogenic bacteria", THE JOURNAL OF MICROBIOLOGY, Bd. 49, Nr. 2, 1. April 2011 (2011-04-01), Seiten 193-199, XP055047024, ISSN: 1225-8873, DOI: 10.1007/s12275-011-0252-9
- DATABASE WPI Week 201039 Thomson Scientific, London, GB; AN 2010-G45646 XP002688915, & JP 2010 124772 A (LION CORP) 10. Juni 2010 (2010-06-10)
- KOLL P . MÄNDAR ET AL: "Characterization of oral lactobacilli as potential probiotics for oral health", ORAL MICROBIOLOGY AND IMMUNOLOGY, MUNKSGAARD, COPENHAGEN, DK, Bd. 23, 1. April 2008 (2008-04-01), Seiten 139-147, XP002594151, ISSN: 0902-0055
- DATABASE WPI Week 201178 Thomson Scientific, London, GB; AN 2011-C10572 XP002688916, & KR 2011 0017534 A (MI C S) 22. Februar 2011 (2011-02-22)

## Beschreibung

Die vorliegende Erfindung betrifft neue Milchsäurebakterien, Analoga oder Fragmente davon, sowie Zusammensetzungen, die diese enthalten, insbesondere zur Verwendung als Probiotikum und/oder in der Dentalhygiene und -therapie. Insbesondere betrifft die vorliegende Erfindung die Verwendung der neuen Milchsäurebakterien bzw. der dieser enthaltenden Zusammensetzungen zur Behandlung und/oder Prophylaxe von Parodontitis, Gingivitis und Periimplantitis in Säugetieren.

Ferner betrifft die vorliegende Erfindung die Verwendung des erfindungsgemäßen Mikroorganismus, Analoges oder Fragmentes davon in Zusammensetzungen oder Nahrungsmitteln oder Tierfutter.

### HINTERGRUND

Bei einem gesunden Erwachsenen besteht zwischen der Flora des gesunden Sulcus, d.h. der um den Zahn verlaufenden Vertiefung zwischen Zahnoberfläche und Zahnfleisch, und den anderen Subhabitaten der Mundhöhle sowie der individuellen Immunantwort und Mundhygiene des Wirts in der Regel ein dynamisches Gleichgewicht. Zum eigentlichen Ausbruch einer Zahnfleischentzündung und Entzündung des Zahnhalteapparats und einer Parodontitis, die zu den häufigsten Erkrankungen des menschlichen Körpers zählen, führt in der Regel eine Verschiebung dieses mikrobiellen Gleichgewichts; begünstigt wird dieser Vorgang dabei durch die Bildung von bakteriellen Plaques (Biofilmen), und einem damit verbundenen gehäuften Auftreten von Gram-negativen pathogenen Spezies innerhalb dieser Ablagerungen. Die von diesen produzierten Exotoxine und bakteriellen Stoffwechselprodukte sammeln sich an und rufen dadurch Entzündungen des umgebenden Zahnfleischgewebes hervor, welche wiederum zu Schwellungen und Blutungen führen. Im weiteren Verlauf der Entzündung wird die Haftung des Saumepithels am Zahn geschwächt, so dass Bakterien in den subgingivalen Bereich vordringen können. Dies gilt analog für die meisten Säugetiere, insbesondere auch für Pferde, Katzen, Nagetiere, Nutztiere und Hunde. Beispielsweise weisen ca. 80 % aller Hunde im Alter von über 3 Jahren peridontale Erkrankungen, insbesondere im Bereich der Eckzähne auf.

Die parodontal-pathogenen Bakterien zeichnen sich durch die Fähigkeit aus, im Parodont die Freisetzung von pro-inflammatorischen Cyotkinen sowie gewebeschädigenden Enzymen und Mediatoren zu veranlassen und dadurch eine entzündliche Parodontolyse herbeizuführen. Ferner besitzen diese pathogenen Bakterien die Fähigkeit, die Immunabwehr zu umgehen, und Leukotoxine sowie Kofaktoren, die gegen sie gerichtete Immunglobuline abbauen und Lymphozytenfunktionen unterdrücken, zu produzieren. Durch die Ausbildung von Schleimkapseln, die bakterielle Lipopolysaccharide maskieren, können sie darüber hinaus einer Phagocytose entkommen. Aber auch durch das aktive Eindringen ins Gingiva-Epithel entziehen sich die Keime der Immunabwehr des Wirtes und hemmen die Chemotaxis von polymorphkernigen neutrophilen Granulozyten (PMN).

Bei dem Übergang von der Gingivitis zur Parodontitis kommt es erneut zu einer deutlichen Veränderung in der Zusammensetzung der mikrobiellen Gemeinschaft. In der nunmehr mehrere Millimeter tiefen Zahnfleischtasche herrschen anaerobe Wachstumsbedingungen. Die mit Parodontitis assoziierten Bakterien sind in der Regel gram-negativ und tragen daher auf ihrer Zellmembran Lipopolysaccharide (LPS), die die primären Liganden des TLR-4 (tolllike receptor) bzw. auch TLR-2 von eukaryontischen Epithelzellen und auch Fibroblasten darstellen. Dieser Rezeptor erkennt und interagiert mit dem LPS als ein LPS-Rezeptor-Protein-Komplex nach Bindung an die Makrophagenzellwand durch das Zelloberflächenmolekül CD14. Durch diese Interaktion wird eine Reihe von Signalmolekülen in der Zelle aktiviert, die eine Freisetzung des Transkriptionsfaktors NF B in den Kern bewirken. Die auf diese Weise stimulierten Makrophagen setzen daraufhin unter anderem Cytokine (IL-1 , TNF-) und Chemokine (IL-8 und MCP-1) frei und damit wird die entzündliche Abwehrreaktion in Gang gesetzt.

Das freigesetzte Interleukin-8 (IL-8) hat eine stark chemotaktische und aktivierende Wirkung auf PMN. Die ausgeschütteten Tumor-Nekrose-Faktor TNF und IL-1 werden an örtliche Fibroblasten gebunden, mit der Folge, dass auch die Fibroblasten PG-E2, MMPs und Interleukin-8 sezernieren. PG-E2 wird von den Fibroblasten in größeren Mengen freigesetzt als von Makrophagen und löst zusammen mit IL-1 , TNF- und IL-6 die entzündliche Knochendestruktion aus. Die Hauptverursacher der aggressiven Zelldestruktion sind die Matrix-Metalloproteinasen (MMPs), die die Proteinbestandteile der extrazellulären Matrix-insbesondere Kollagen - enzymatisch abbauen. Typ I Kollagen bildet die dominierende Kollagenklasse im humanen Parodont. MMP-1 besitzt die Fähigkeit, interstitielle Kollagenfasern, welche normalerweise gegen Proteolyse resistent sind, abzubauen. MMP-8 ist noch potenter als MMP-1, vor allem gegen Kollagen-I. MMP-8 spielen *in vivo* eine wesentliche Rolle bei der entzündlichen Parodontolyse und mit über 90 % den Hauptteil der Kollagenaseaktivität aus.

Subgingivale Biofilme sind sehr widerstandsfähig und können nicht durch häusliche Pflege, auch nicht durch Spülungen oder durch die Anwendung antimikrobiell wirkender Zahnpasten oder Mundwässern eliminiert werden. Vielmehr ist die lokale mechanische Entfernung oder Zerstörung erforderlich. Die pathogenen Spezies sind in der Regel auch in Plaques gesunder Personen nachweisbar. Erst die Verschiebung des mikrobiellen Gleichgewichts verbunden mit der Zunahme der pathogenen Spezies innerhalb der Plaque führt zum Ausbruch der Zahnfleischentzündung und der Parodontitis. Dementsprechend sollte die Prophylaxe einer Parodontitis möglichst in einem Stadium einsetzen, in dem nur wenige pathogene Keime die Mundhöhle besiedeln und sich noch keine Entzündung manifestiert hat.

### STAND DER TECHNIK

Zur Parodontitis-Therapie wird gegenwärtig üblicherweise die subgingivalen Plaque zunächst mechanisch durch Abkratzen oder mittels Ultraschall entfernt; durch diese mechanische Beseitigung werden die Bakterien im Biofilm herausgelöst und herausgespült. Im Zuge dieser Behandlung werden heutzutage auch Antibiotika sowie andere bakterizide Zusammensetzungen eingesetzt, die die erneute Besiedelung durch gram-negative Bakterien verhindern sollen. Nachteilig bei einem regelmäßigen und extensiven Einsatz von Zusammensetzungen, die Antibiotika enthalten, ist jedoch die Gefahr der Entwicklung von Antibiotika-Resistenzen und der oftmals ausgelösten Nebenwirkungen dieser Produkte.

Daher besteht nach wie vor ein großes Bedürfnis an wirksamen und gut verträglichen Mitteln zur Behandlung und/oder Prophylaxe von Parodontitis und Gingivitis, sowie an der Identifizierung von Mitteln, die als Probiotikum Nahrungsmitteln zugesetzt werden können.

Die Aufgabe der vorliegenden Erfindung ist es daher, einfache Mittel bereitzustellen, die in der Dentalhygiene und -therapie eingesetzt werden können, ohne schädliche Nebenwirkungen hervorzurufen.

Teughels et al., "Probiotics and oral healthcare", Periodontology 2000, 2008, 48:111-147, beschreiben dass bestimmte Milchsäurebakterien *Porphyromonas gingivalis* generell abtöten konnten.

Darüberhinaus beschreiben Bonifait et al., "Probiotics for oral health: Myth or rality", Journal Canadian Dental Association, 2009, 75(8):585-590, Koduganti et al., "Probiotics and prebiotics in periodontal therapy", Indian Journal of Dental Research, 2011, 22:324-330, Teughels et al., "Bacteria Interfere with A. actinomycetemcomitans Colonization", Journal of Dental Research, 2007, 86(7):611-617, die WO 2006/022471 A1, Teanpaisan et al., "Inhibitory effect of oral Lactobacillus against oral pathogens", Letters in Applied Microbiology, 2011, 53(4):452-459, Kang et al., "Inhibitory Effect of Lactobacillus reuteri on Periodontopathic and Cariogennic Bacteria"; Journal of Microbiology, 2011, 49(2): 193-199, Köll et al., "Characterization of oral lactobacilli as potential probiotics for oral health", Oral Microbiology Immunology, 2008, 23:139-147, und ferner die US 2003/0077814 A1 die generelle Eignung von Lactobacillen, das Wachstum bzw. die Besiedelung von bestimmten oralpathogenen Bakterien zu inhibieren.

Ferner offenbart die WO 2005/120527 A1 ein Verfahren zur Auswahl eines Milchsäurebakterien-Stammes, der anti-mikrobielle und anti-entzündliche Eigenschaften aufweist, der ferner an Mucin bindet und Vitamin K produziert.

### DARSTELLUNG DER ERFINDUNG

Erfindungsgemäß wird diese Aufgabe gelöst durch einen Mikroorganismus, der zur Gattung der Milchsäurebakterien gehört, oder ein Fragment davon gemäß den beigefügten Ansprüchen, wobei der Mikroorganismus oder das Fragment davon u.a. die Fähigkeit zur spezifischen Bindung an zumindest einem oralpathogenen Mikroorganismus aufweist, welcher ausgewählt ist aus der Gruppe *Porphyromonas sp., Treponema sp.* oder *Actinobacillus sp..* Dabei besitzt der Mikroorganismus, der zur Gattung der Milchsäurebakterien gehört, eine natürliche Fähigkeit zur spezifischen Bindung von zumindest einem oralpathogenen Mikroorganismus, was bedeutet, dass diese Fähigkeit dem Mikroorganismus von Natur aus innewohnt, ohne dass diese durch eine gentechnische Modifikation des zur Gattung der Milchsäurebakterien gehörenden Mikroorganismus herbei geführt wurde. In diesem Zusammenhang bedeutet also vorliegend erfindungsgemäß ein Mikroorganismus, der zur Gattung der Milchsäurebakterien gehört, dass das Genom des zur Gattung der Milchsäurebakterien gehörenden Mikroorganismus nicht mittels gentechnischer Methoden gezielt zur spezifischen Bindung der oralpathogenen Mikroorganismen verändert wurde, der beanspruchte Mikroorganismus ist in dieser Hinsicht also gentechnisch unmodifiziert.

Ein weiterer Aspekt der vorliegenden Erfindung betrifft ein Fragment des beschrieben Mikroorganismus gemäß den beigefügten Ansprüchen, welches hergestellt wurde durch thermische Inaktivierung oder Lyophilisation, wobei das Fragment auch nach der thermischen Inaktivierung oder nach Lyophilisation die Fähigkeit zur spezifischen Bindung von zumindest einem oralpathogenen Mikroorganismus aufweist, welcher aus der Gruppe *Porphyromonas sp., Treponema sp.* oder *Actinobacillus sp.* ausgewählt ist. Ferner betrifft die vorliegende Erfindung auch Zusammensetzungen gemäß den beigefügten Ansprüchen, die den erfindungsgemäßen Mikroorganismus enthalten, oder Fragmente davon, welche die Fähigkeit zur spezifischen Bindung der oben aufgeführten oralpathogenen Mikroorganismen aufweisen, und die insbesondere als Nahrungsmittel, Nahrungsmittelzusatz, als Tiernahrung oder Getränke eingesetzt werden.

Die Aufgabe wird ferner gelöst durch eine Zusammensetzung gemäß den beigefügten Ansprüchen, die den erfindungsgemäßen Mikroorganismus oder Fragmente davon enthält, welche die Fähigkeit zur spezifischen Bindung mindestens eines oralpathogenen Mikroorganismus aufweisen, der ausgewählt ist aus der Gruppe *Porphyromonas sp., Treponema sp.* oder *Actinobacillus sp.,* für die Dentalhygiene,therapie und/oder -prophylaxe. Dabei ist bevorzugt, wenn es sich bei den oralpathogenen Mikroorganismen um *Porphyromonas gingivalis, Treponema denticola* oder *Actinobacillus actinomycetemcomitans* handelt.

Die der Erfindung zugrunde liegende Aufgabe wird dadurch vollkommen gelöst.

Die erfindungsgemäßen Mikroorganismen sind Vertreter der Gattung der Milchsäurebakterien, mithin also Gram-positive Bakterien, die Milchsäure durch Fermentation von Glucose erzeugen. Der erfindungsgemäße Mikroorganismus zeichnet sich einerseits dadurch aus, dass er die Fähigkeit zur spezifischen Bindung von zumindest einem oralpathogenen Mikroorganismus aufweist, der ausgewählt ist aus der Gruppe *Porphyromonas sp., Treponema sp.* oder *Actinobacillus sp.* Diese Bindung führt zu einer Bildung von Aggregaten aus den erfindungsgemäßen Mikroorganismen und den spezifisch gebundenen, oralpathogenen Mikroorganismen. Durch die Aggregat-Bildung können letztere, also die oralpathogenen Mikroorganismen mechanisch, bspw. durch Ausspülen, gezielt und leicht entfernt werden, was durch bisher bekannte Maßnahmen nicht möglich war. Damit kann wiederum die Zahl der parodontal-/oral-pathogenen Mikroorganismen in der Zahnfleischtasche, sowie der durch diese Mikroorganismen gebildete Biofilm gehemmt werden. Des Weiteren wird durch Maskierung der Oberflächenstrukturen oralpathogener Mikroorganismen eine initiale bzw. erneute Anheftung an Zähne und umgebendes Gewebe verhindert bzw. reduziert.

Unter dem Ausdruck "spezifische Bindung" oder "Aggregation" wird vorliegend sowie üblicherweise im Gebiet der Mikrobiologie, insbesondere der oralen Mikrobiologie und Dentalhygiene die gegenseitige Erkennung und Adhäsion von Zellen, die genetisch unterschiedlichen Typen von Zellen angehören, verstanden. Hierzu exprimieren Bakterien auf ihrer Zelloberfläche Rezeptoren und Strukturen für Adhäsine auf anderen Zelltypen, die für die Anheftung zwischen den Zellen verwendet werden. Diese Adhärenz spielt für die Besiedelung mit pathogenen wie auch mit kommensalen Mikroorganismen eine herausragende Rolle, so dass ein Eingriff in die Adhärenz zu weitreichenden Konsequenzen führen kann. Dadurch, dass die erfindungsgemäßen Mikroorganismen die Fähigkeit zur spezifischen Bindung und Aggregation mit zumindest einem der weiter oben aufgeführten oralpathogenem Mikroorganismen aufweisen, können sich im Falle einer Behandlung des Mundraumes eines Patienten mit den erfindungsgemäßen Mikroorganismen Aggregate aus den erfindungsgemäßen Mikroorganismen und den aufgeführten oralpathogenen Mikroorganismen bilden, und diese entstehenden Co-Aggregate können leicht, bspw. durch Spülen, aus dem Mund entfernt werden.

Vorliegend wird ferner unter "spezifische Bindung von zumindest einem oralpathogenen Mikroorganismus" die Eigenschaft des erfindungsgemäßen Mikroorganismus verstanden, mindestens einen von *Porphyromonas sp., Treponema sp.* oder *Actinobacillus sp* spezifisch zu binden, oder aber zumindest zwei, d.h. *Porphyromonas sp.* und *Treponema sp.,* oder *Porphyromonas sp.* und *Actinobacillus sp.,* oder *Treponema sp.* und *Actinobacillus sp.,* oder aber alle drei, *Porphyromonas sp., Treponema sp*. und *Actinobacillus sp.*

Vorliegend wird unter "gentechnisch unmodifizierter" Mikroorganismus ein Mikroorganismus verstanden

Gemäß einer bevorzugten Ausführungsform der erfindungsgemäßen Mikroorganismen zeichnen sich diese ferner dadurch aus, dass die Fähigkeit zur spezifischen Bindung an zumindest einen oralpathogenen Mikroorganismus auch nach einer Hitzebehandlung bei mind. 70 °C besteht.

Unter der genannten Hitzebehandlung bzw. unter "resistent gegenüber einer Hitzebehandlung" oder "Hitzestabilität" wird dabei die Eigenschaft eines Bakteriums verstanden, auch nach einem bestimmten, längeren Zeitraum von mindestens 30 min bei einer erhöhten Temperatur von 70 °C oder mehr noch in der Lage zu sein, eine spezifische Bindung mit zumindest einem oralpathogenen Mikroorganismus einzugehen, das aus der Gruppe *Porphyromonas sp., Treponema sp.* oder *Actinobacillus sp.* ausgewählt ist.

Gemäß dieser weiteren erfindungsgemäßen Eigenschaft können sich die erfindungsgemäßen Mikroorganismen daher, zusätzlich zu der oben beschriebenen Eigenschaft der Fähigkeit zur spezifischen Bindung mindestens eines oralpathogenen Mikroorganismus, der aus der Gruppe *Porphyromonas sp., Treponema sp.* oder *Actinobacillus sp.* ausgewählt ist, auch durch Hitzestabilität auszeichnen, und eine Behandlung mit hohen Temperaturen, vorzugsweise von mindestens etwa 60 °C, bevorzugter von mindestens 65 °C und noch bevorzugter von mindestens 70 °C, über einen Zeitraum von mindestens 20 Minuten, vorzugsweise von 25 Minuten und noch bevorzugter von mindestens ca. 30 Minuten, überstehen und bezüglich ihrer Fähigkeit zur spezifischen Bindung von zumindest einem der genannten oralpathogenen Mikroorganismen unverändert bleiben.

Gemäß einer weiteren bevorzugten Ausführungsform weist der erfindungsgemäße Mikroorganismus auch die Eigenschaft auf, dass die Fähigkeit zur spezifischen Bindung an den zumindest einen erwähnten oralpathogenen Mikroorganismus auch in Gegenwart von natürlichem Speichel besteht.

Natürlicher Speichel enthält u.a. antimikrobielle Substanzen, wie bspw. Lysozym, das der Abwehr von Bakterien dient, und das die Zellwand von insbesondere Gram-positiven Bakterien direkt angreifen kann. Die erfindungsgemäßen Mikroorganismen zeichnen sich hingegen dadurch aus, dass sie trotz des Vorliegens von natürlichem Speichel und trotz der darin enthaltenen antimikrobielle Stoffe immer noch zur spezifischen Bindung an die erwähnten oralpathogenen Mikroorganismen befähigt sind. Die Bindung des erfindungsgemäßen Mikroorganismus an die aufgeführten oralpathogenen Mikroorganismen führt zu einer Hemmung des Wachstums dieser oralpathogenen Mikroorganismen.

Ferner ist bevorzugt, wenn die Fähigkeit der erfindungsgemäßen Mikroorganismen zur spezifischen Bindung an die oralpathogenen Mikroorganismen auch bei einem pH-Wert von zwischen ca. 2,5 und 9 besteht.

Dem Fachmann wird hierbei, sowie bei sämtlichen in der vorliegenden Erfindung aufgeführten Bereichsangaben, die durch die Begriffe "ca. " oder "etwa" gekennzeichnet sind, klar sein, dass mit dem Ausdruck "ca. " oder "etwa" nicht die exakte Zahlenangabe gemeint sein muss, sondern dass auch geringfügige Abweichungen nach oben oder unten bezüglich der angegebenen Zahl noch in den Rahmen der vorliegenden Erfindung liegen. Derartige Abweichungen können bspw. durch den Einsatz unterschiedlicher Messverfahren/-instrumente zustande kommen und Toleranzen darstellen, die der Fachmann ohne Weiteres erfasst und als mögliche, für die vorliegende Erfindung anwendbare Werte in Betracht zieht.

Ferner zeichnen sich die erfindungsgemäßen Mikroorganismen auch dadurch aus, dass deren Bindungsfähigkeit zu einem Aggregat aus Mikroorganismus und pathogenen Keim führt, das sich bei einer Zentrifugalkraft von 300 x g über 30 Sekunden abzentrifugieren lässt.

Gemäß einer weiteren bevorzugten Ausführungsform besitzt der erfindungsgemäße Mikroorganismus nicht die Fähigkeit, an *Streptococcus salivarius* zu binden.

*Streptococcus salivarius* stellt ein weitestgehend unauffälliger Kommensale der Mundhöhle dar, der hauptsächlich von Schleimhautoberflächen des Mundvorhofs und der Zunge isoliert werden kann. Im Zusammenspiel der unterschiedlichen, in der Mundhöhle vorhandenen Mikroorganismen ist er in der gesunden oralen Mikroflora vieler Säugetiere vorhanden, und steht - zumindest bei gesunden Zähnen - mit diesen im mikrobiellen Gleichgewicht. Daher ist eine Beeinflussung dieses Bakteriums, bspw. durch eine Aggregation und dadurch Beeinflussung mit anderen Mikroorganismen, die gezielt in der Dentalhygiene oder Dentaltherapie eingesetzt werden sollen, im Rahmen der vorliegenden Erfindung nicht erwünscht.

Erfindungsgemäß hemmt der erfindungsgemäße Mikroorganismus die Bildung eines Biofilms, der von zumindest einem der genannten oralpathogenen Mikroorganismen gebildet wird.

*Porphyromonas gingivalis* und *Treponema denticola,* sowie auch *Actinobacillus actinomycetemcomitans* sind anerkanntermaßen drei Vertreter von oralpathogenen Bakterien, die bei der Entwicklung von Parodontitis, Gingivitis und Periimplantitis eine zentrale Rolle spielen. Diese pathogenen Bakterien bilden Biofilme, die, wie eingangs erläutert, Auslöser für die zuvor erwähnten Dentalerkrankungen sind. Diese pathogenen Bakterien setzen sich am Zahn/in den Zahntaschen fest und produzieren extrazelluläre Schleime und Polysaccharide und bilden mit aus Speichel adsorbierten Proteinen und Glykoproteinen einen sogenannten Biofilm (auch Plaque genannt). Die beiden erwähnten pathogenen Bakterien produzieren in dem Biofilm Toxine, die zu chronischen Entzündungen und der Zerstörung des periodentalen Ligaments und sogar des benachbarten alveolären Knochens führen können. Die hierdurch ausgelösten Erkrankungen wie Parodontitis, Gingivitis und Periimplantitis können durch die Hemmung der Biofilmbildung der genannten oralpathogenen Mikroorganismen durch die erfindungsgemäßen Mikroorganismen erfolgreich behandelt bzw. deren Entstehung verhindert werden.

Die vorliegende Erfindung betrifft ferner einen Mikroorganismus, oder ein Fragment davon gemäß den beigefügten Ansprüchen, wobei der Mikroorganismus zur Gattung der Milchsäurebakterien gehört, und wobei der Mikroorganismus ferner nicht über die Fähigkeit, an *Streptococcus salivarius* zu binden verfügt, sowie ggf. auch Hitzestabilität aufweist.

Die erfindungsgemäßen Mikroorganismen zeichnen sich dadurch aus, dass sie an zumindest einen oralpathogenen Mikroorganismus binden, der ausgewählt ist aus der Gruppe *Porphyromonas sp., Treponema sp., Actinobacillus sp,* und insbesondere *Porphyromonas gingivalis* bzw. *Treponema denticola,* wobei die erfindungsgemäßen Mikroorganismen die genannten Bakterienstämme spezifisch binden und aggregieren, und dadurch deren Biofilm-Bildung hemmen.

Vorliegend besitzen gemäß einer bevorzugten Ausführungsform die erfindungsgemäßen Mikroorganismen die Eigenschaft, hitzeresistent zu sein, d.h. auch noch nach einer Hitzebehandlung wie oben geschildert, vorzugsweise bei 70 °C für mind. 20 min, die Fähigkeit zu einer spezifischen Bindung an oralpathogene Mikroorganismen zu besitzen. Gemäß einer weiteren Alternative besitzt der erfindungsgemäße Mikroorganismus die Eigenschaft, sowohl an *Porphyromonas sp.,* vorzugsweise *Porphyromonas gingivalis,* als auch an *Treponema sp.,* vorzugsweise *Treponema denticola,* zu binden, also zu aggregieren, wodurch es zu einer Aggregat-Bildung mit diesen kommt.

Eine weitere Eigenschaft der erfindungsgemäßen Mikroorganismen ist, wie bereits weiter oben erwähnt, deren Fähigkeit zur Hemmung der Biofilmbildung durch *Porphyromonas gingivalis, Treponema denticola* oder *Actinobacillus actinomycetemcomitans.* Diese Bakterien spielen nachgewiesenermaßen bei der Parodontitis eine große Rolle. Sie bilden extrem zähe Biofilme, die gegenüber Ultraschall, Detergentien, Proteasen, Hitze resistent sind, und sich auch schon als resistent gegenüber antimikrobiellen Substanzen erwiesen haben. Der erfindungsgemäße Mikroorganismus besitzt daher die Eigenschaft, die Biofilm-Bildung durch *Porphyromonas gingivalis, Treponema denticola* oder *Actinobacillus actinomycetemcomitans* zu hemmen; aufgrund der Hemmung der Biofilm-Bildung können diese pathogenen Bakterien die Mundhöhle nicht mehr besiedeln, und können konsequenterweise auch keine Erkrankungen wie Parodontitis, Periimplantitis und Gingivitis auslösen.

Daher wird vorliegend unter "Hemmung der Biofilm-Bildung durch *Porphyromonas sp.,* insbesondere *Porphyromonas gingivalis, Treponema sp.,* insbesondere *Treponema denticola* oder *Actinobacillus sp.,* insbesondere *Actinobacillus actinomycetemcomitans*" jede Eigenschaft eines Mikroorganismus verstanden, derart mit *Porphyromonas sp.,* insbesondere *Porphyromonas gingivalis, Treponema sp.,* insbesondere *Treponema denticola* oder *Actinobacillus sp.,* insbesondere *Actinobacillus actinomycetemcomitans* zu interagieren, d.h. an diese zu binden, so dass diese keinen Biofilm mehr bilden können.

Gemäß einer bevorzugten Ausführungsform kann der erfindungsgemäße Mikroorganismus neben der Fähigkeit zur spezifischen Bindung an zumindest einen, vorzugsweise zwei, oralpathogene Mikroorganismen und der Fähigkeit zur Hemmung der Biofilm-Bildung durch *Porphyromonas sp., Treponema sp.* oder *Actinobacillus sp.* auch Hitzeresistenz besitzen.

Mit dem Ausdruck "Mikroorganismus, der zu der Gattung der Milchsäurebakterien gehört" werden vorliegend, wie bereits aufgeführt, auch Derivate oder Analoga oder Fragmente davon umfasst, welche die hierin beschriebenen Charakteristika bzw. Merkmale oder Eigenschaften der erfindungsgemäßen Mikroorganismen noch besitzen. Vorzugsweise handelt es sich bei den erfindungsgemäßen Milchsäurebakterien um Bakterien der Art *Lactobacillus gasseri, Lactobacillus crispatus* und *Lactobacillus acidophilus.*

Beschrieben werden hierin auch "ein Analogon oder ein Derivat" der zuvor erwähnten Mikroorganismen, die zur Gattung der Milchsäurebakterien gehören, insbesondere ein Analogon oder ein Derivat der im Rahmen der vorliegenden Anmeldung hinterlegten *Lactobacilli sp.,* noch die gleichen Charakteristika wie vorliegend für die beanspruchten Milchsäurebakterien beansprucht, und insbesondere die gleichen wie die hinterlegten Stämme; dies wären zumindest die Fähigkeit zur spezifischen Bindung mindestens eines oralpathogenen Mikroorganismus ausgewählt aus der Gruppe *Porphyromonas sp., Treponema sp.* oder *Actinobacillus sp.* und die Fähigkeit zur Hemmung der Bildung eines Biofilms des zumindest einen oralpathogenen Mikroorganismus, sowie ggf. noch zumindest eines, zwei oder drei der folgenden Merkmale (i) Resistenz der Fähigkeit zur spezifischen Bindung gegenüber einer Hitzebehandlung bei mehr als 70 °C für mindestens 30 min; (ii) keine Bindung an *Streptococcus salivarius;* (iii) Zustandekommen der spezifischen Bindung bei pH 2,5 - 9. Solche Derivate können bspw. gentechnologisch hergestellt werden. Dabei beinhaltet vorliegend der Ausdruck "genetechnologisch hergestellt" im weitesten Sinne alle Verfahren, die dem Fachmann im Gebiet der Gentechnologie bekannt sind, um Nukleinsäuren *in vitro* und *in vivo* derart zu modifizieren, dass durch rekombinante DNA-Technologien genetische Modifizierungen bewirkt und Gene verändert werden können.

Entsprechend umfasst die vorliegende Erfindung auch Fragmente der erfindungsgemäßen Mikroorganismen, welche die weiter oben und in den Ansprüchen definierten Eigenschaften der erfindungsgemäßen Mikroorganismen noch besitzen. In diesem Zusammenhang versteht es sich dann, dass anstelle der Mikroorganismen die Fragmente davon im Sinne der vorliegenden Erfindung in der Dentalhygiene, -therapie und -prophylaxe eingesetzt werden. Ein "Fragment" im Sinne der vorliegenden Erfindung ist dabei jeder Teil der Zellen der erfindungsgemäßen Mikroorganismen, und vorzugsweise ein Teil der Zellmembran. Die Gewinnung von Zellmembran-Fraktionen ist dem Fachmann aus dem Stand der Technik hinreichend bekannt (siehe bspw. Sambrook und Rüssel: Molecular Cloning, A Laboratory Manual, dritte Auflage).

Die erfindungsgemäßen Mikroorganismen liegen dabei vorzugsweise isoliert oder gereinigt vor, wobei der Begriff "isoliert" vorliegend bedeutet, dass die Milchsäurebakterien ihrem Kultivierungsmedium/-milieu - auch bspw. ihrem natürlichen - entnommen sind. Der Ausdruck "gereinigt" muss sich vorliegend nicht auf eine absolute Reinheit beziehen.

Ferner versteht sich, dass neben den erfindungsgemäßen Mikroorganismen in lebensfähiger Form auch inaktive Formen der erfindungsgemäßen Mikroorganismen vom Rahmen der vorliegenden Erfindung umfasst sein. Dabei bedeutet "inaktive Form" inaktivierte oder tote Zellen, die nicht mehr zur Koloniebildung auf Kultivierungsplatten in der Lage sind. Geeignete Verfahren zur Inaktivierung sind dem Fachmann hinreichend bekannt. Vorliegend können aber die Mikroorganismen auch in lyophilisierter Form eingesetzt werden. Lyophilisierte Zellen können nach geeigneter Kultivierung in flüssigen oder festen Medium wieder zum Anwachsen gebracht werden.

Die Begriffe "inaktivierte Formen" und "Derivate" oder "Analoga" schließen vorliegend auch Lysate, Fraktionen oder Extrakte der erfindungsgemäßen Mikroorganismen mit ein, wobei diese Lysate, Fraktionen oder Extrakte erfindungsgemäß die oben erwähnten Eigenschaften der vorliegend beschriebenen Mikroorganismen besitzen. Hierbei bedeutet "Lysat" - wie auch der Begriff "Extrakt" - eine Lösung oder Suspension in einem wässrigem Medium der erfindungsgemäßen Zellen des Mikroorganismus, und umfasst bspw. Makromoleküle, wie DNA, RNA, Proteine, Peptide, Lipide u.ä., sowie auch Zelltrümmer. Vorzugsweise umfasst das Lysat auch Zellwand oder Zellwandbestandteile. Verfahren zur Herstellung von Lysaten sind dem Fachmann hinreichend bekannt und schließen den Einsatz einer "French Press", enzymatische Lyse u.ä. mit ein.

Ob ein Mikroorganismus, der zur Gattung der Milchsäurebakterien gehört, die erfindungsgemäßen Eigenschaften aufweist, kann vom Fachmann bspw. anhand der vorliegend und nachstehend noch beschriebenen Versuche getestet und überprüft werden.

Der erfindungsgemäße Mikroorganismus ist ausgewählt aus einem der folgenden Mikroorganismen, die jeweils unter den Hinterlegungsnummern DSM 25257, DSM 25253, DSM 25254 und DSM 25255 jeweils am 07.Oktober 2011 bei der Deutschen Sammlung für Mikroorganismen und Zellkulturen (DSMZ) in Braunschweig vorschriftsmäßig hinterlegt wurden.

Die erwähnten DSMZ-Hinterlegungen wurden gemäß dem Budapester Vertrag über die internationale Anerkennung der Hinterlegung von Mikroorganismen zum Zwecke der Patenthinterlegung vorgenommen.

Wie weiter oben erwähnt, betrifft die vorliegende Erfindung auch Zusammensetzungen, enthaltend lebensfähige Mikroorganismen oder ein Fragment davon wie in den beigefügten Ansprüchen definiert, sowie zumindest einen Träger oder Exzipienten, der gemäß einer bevorzugten Ausführungsform bspw. ausgewählt ist aus zumindest einem der folgenden: einen kosmetisch verträglichen Träger oder Exzipienten, einem pharmazeutisch verträglichen Träger oder Exzipienten oder einem oral verträglichen Träger oder Exzipienten.

Dabei wird unter einer "Zusammensetzung" im Sinne der vorliegenden Erfindung jede Zusammensetzung verstanden, die zumindest einen erfindungsgemäßen Mikroorganismus aufweist, oder ein Fragment davon, sowie ggf. weitere Inhaltsstoffe wie Träger oder Hilfsstoffe, oder ggf. noch weitere aktive Inhaltsstoffe und Salze. Der Träger/Exzipient ist dabei je nach Einsatz bzw. Anwendungsbereich der erfindungsgemäßen Zusammensetzung ausgewählt; diese kann insbesondere kosmetisch, pharmazeutisch oder oral verträgliche Träger/Exzipienten enthalten. Unter kosmetisch, pharmazeutisch oder oral verträglichen Hilfsstoffen, Trägern oder Exzipienten wird dabei jede Substanz oder Stoff verstanden, der jeweils üblicherweise im kosmetischen, pharmazeutischen oder im Dentalbereich eingesetzt wird, um einen aktiven Inhaltsstoff der Zusammensetzung, vorliegend also der zumindest einen Mikroorganismus, oder Analogon oder Derivat oder Fragment davon, jeweils kosmetisch, pharmazeutisch oder oral einzusetzen, zu verabreichen und zur Wirkung zu bringen.

Es versteht sich dabei vorliegend, dass die erfindungsgemäße Zusammensetzung nicht nur einen der erfindungsgemäßen Mikroorganismen, oder Fragment davon aufweisen kann, sondern auch eine Mischung von erfindungsgemäßen Mikroorganismen oder eine Mischung der Fragmente, oder aber eine Mischung aus den erfindungsgemäßen Mikroorganismen und den Fragmenten.

Die Zusammensetzung kann dabei in fester oder flüssiger oder viskoser Form oder als Aerosol vorliegen, und kann u.a. in Form von Pulvern, Tabletten, Lösungen, Granulaten, Suspensionen, Emulsionen, Kapseln, Pasten, Gelen, Sprays, etc. vorliegen, mithin in jeder Form, die für eine orale Verabreichung geeignet ist. Ferner können, je nach Einsatz, auch weitere Probiotika, Antiseptika oder andere antibakteriell wirksame Substanzen in der Zusammensetzung enthalten sein, sowie ggf. Saccharide und Konservierungsmittel, Geschmacksstoffe, Süßstoffe, Vitamine, Mineralien, etc. Die EP 2 133 414 A1 listet eine Reihe von Inhaltsstoffen auf, die für derartige Zusammensetzungen eingesetzt werden können; auf diese Veröffentlichung wird hiermit explizit Bezug genommen. Außerdem können Füllstoffe, Fließmittel, Rheologiemodifikatoren, Weichmacher, Stabilisatoren, Initiatoren oder auch reaktiv vernetzende Monomere wie Methacrylate o.ä. in den Zusammensetzungen vorhanden sein.

Die erfindungsgemäße Zusammensetzung wird dabei insbesondere zur Verwendung in der Dentalhygiene, -therapie und -prophylaxe eingesetzt, und weist zumindest einen erfindungsgemäßen Mikroorganismus oder Derivate/Fragmente/Analoga davon auf.

Die Zusammensetzung bzw. der Mikroorganismus kann ferner auch als Nahrung, Futtermittel, Futtermittelzusatz, Nahrungsergänzungsmittel eingesetzt werden, deren Einnahme dann als Prophylaxe dient. Auch diesbezüglich wird explizit auf die in der EP 2 133 414 A1 veröffentlichten Möglichkeiten zum Einsatz einer Mikroorganismen - bzw. Fragmente davon - enthaltenden Zusammensetzung Bezug genommen.

Insbesondere ist die vorliegende erfindungsgemäße Zusammensetzung als Probiotikum geeignet, also als eine Zubereitung, die lebensfähige Mikroorganismen enthält, und die oral eingenommen eine gesundheitsfördernden Einfluss auf denjenigen haben, der das Probiotikum einnimmt bzw. dem dieses verabreicht wird. Derartige Zubereitungen können Nahrungsmittel oder Nahrungsmittelzusätze oder Futtermittel oder Futtermittelzusätze oder aber Medikamente sein. Erfindungsgemäß kann die Zusammensetzung bei allen Säugetieren eingesetzt werden, also außer beim Menschen auch bei Hund, Katze, Rind, Pferd, Affen, Schafen, Ziegen, Schwein, Meerschweinchen, Hamster, Ratte, Hase, Maus, Frettchen, Chinchilla u.a. Insbesondere kann die erfindungsgemäße Zusammensetzung in/als Tierfutter eingesetzt werden.

Die Dosierung und die Verabreichung an sich, mit welcher die erfindungsgemäße Zusammensetzung eingesetzt wird, wird vom jeweiligen Einsatz/vom jeweiligen Patienten - insbesondere von dessen Alter, Gewicht, dem allgemeinen Zustand, etc. - abhängen und liegt im Können und der Einschätzung des Fachmanns, der die Zusammensetzung zum Einsatz bringt.

Dabei kann die erfindungsgemäße Zusammensetzung kosmetisch, als Medizinprodukt vorliegen oder pharmazeutisch sein, und den Mikroorganismus in einer Menge von zwischen 0,001 Gewichtsprozent bis 90 Gewichtsprozent, bevorzugt bis 70 Gewichtsprozent, besonders bevorzugt bis 40 Gewichtsprozent und ganz besonders bevorzugt bis 25 Gewichtsprozent enthalten. Es versteht sich, dass für spezifische Anwendungen aber auch andere Mengen eingesetzt werden können, die sich von den hierin angegebenen unterscheiden.

Wie weiter oben erwähnt, kann gemäß einer bevorzugten Ausführungsform die Zusammensetzung bzw. der Mikroorganismus im Bereich der Dentalhygiene eingesetzt werden, um die genannten oralpathogenen Keime zu aggregieren, d.h. zu binden. Diese Aggregate können dann leicht entfernt, bspw. bei einer Suspension: ausgespült, werden, wodurch vorteilhaft eine Reduktion der pathogenen Keime bewirkt wird. Die Mikroorganismen bzw. eine diese enthaltende Zusammensetzung kann hierzu unterschiedlich eingesetzt werden. Bspw. können sie auf Einleg-Fäden, auf Zahnseide oder allgemein in Mundspüllösungen oder in Zahnpflegemitteln wie Zahnpasten, Zahnpulvern, Kaugummis, Lutschtabletten oder -bonbons angewendet werden. Besonders bevorzugte weitere Ausführungsformen können Strahlpulver, Polierpasten, Zahnlacke, Fluoridierungsmittel, Desensibilisier, Fissurenversiegler, Retraktionsmittel, Haftvermittler oder auch Füllmaterialien sein. Daher betrifft die vorliegende Erfindung auch sämtliche Produkte, die im Bereich der Denthaltherapie und bevorzugt Dentalprophylaxe eingesetzt werden, und die die erfindungsgemäßen Mikroorganismen enthalten. Oben beschriebene Ausführungsformen gelten entsprechend für den Bereich der Behandlung, Therapie und Prophylaxe bei Säugetieren. Die Mikroorganismen bzw. eine diese enthaltende Zusammensetzung kann hierzu unterschiedlich eingesetzt werden. Bspw. können sie auf oder in Kauknochen, Kausticks, Tiersnacks, Tierfutter, Pellets, Beiß- und Nagekörper (aus Material zum Verzehr geeignet, beispielsweise Pansenteile, Ochsenziemer etc. oder nicht zum Verzehr geeignet, beispielsweise Gummi, Kautschuk, Plastik usw.), Tierspielzeug etc. gebracht bzw. angewendet werden.

Insbesondere ist dabei bevorzugt, wenn die erfindungsgemäße Zusammensetzung und/oder der erfindungsgemäße Mikroorganismus zur Therapie oder Prophylaxe von Parodontitis, Periimplantitis und Gingivitis in Säugetieren, insbesondere beim Menschen, verwendet wird.

Parodontitis, Periimplantitis und Gingivitis sind Erkrankungen des Zahnapparates, wobei sich Parodontitis durch einen Knochenabbau und Gingivitis sich durch vertiefte Zahnfleischtaschen auszeichnen, verursacht durch Entzündungen des Zahnfleisches. Periimplantitis ähnelt der Parodontitis des natürlichen Zahnes, wobei auch die Periimplantitis mit der Entzündung und dem Rückgang von Schleimhaut und Knochen im Bereich eines Implantats verbunden ist. Unbehandelt führt die Periimplantitis üblicherweise zum Verlust des Implantats.

Durch die erfindungsgemäßen Mikroorganismen und die diese enthaltende Zusammensetzung werden Mittel bereit gestellt, mit welchen diese Erkrankungen vorteilhaft behandelt, bzw. vermieden werden können. Die Mikroorganismen bzw. die diese enthaltenden Zusammensetzungen können dabei sowohl in der Human- als auch in der Veterinärmedizin, insbesondere, wie bereits aufgeführt, bei Hund, Katze, Rind, Pferd, Affen, Schafen, Ziegen, u.a. Säugetieren eingesetzt werden.

Wie weiter oben erwähnt kann die erfindungsgemäße Zusammensetzung bzw. die Mikroorganismen - oder Fragmente davon - als Nahrungsmittelzusatz, als Hygieneprodukt, bzw. als ein die Mikroorganismen aufweisendes Hygieneprodukt, oder als eine pharmazeutische Präparation eingesetzt werden. Solche Hygieneprodukte können bspw. auch Kits sein, die neben Dental-/Oralhygienischen Vorrichtungen oder Geräten, Spülungen, Pasten u.ä. auch die erfindungsgemäßen Mikroorganismen oder die diese enthaltenden Zusammensetzungen enthalten.

Die Erfindung betrifft damit ferner auch die Verwendung des erfindungsgemäßen Mikroorganismus zur Therapie und/oder Prophylaxe von Parodontitis, Periimplantitis und Gingivitis.

Die vorliegende Erfindung betrifft ferner ein Verfahren zur Identifizierung und/oder Selektion eines Mikroorganismus der Gattung *Lactobacillus sp.* mit der/den wie in den Ansprüchen definierten erfindungsgemäßen Eigenschaft/en, wobei das Verfahren zumindest die folgenden Schritte aufweist: a) Inkubieren eines Ansatzes aus einem oralpathogenen Mikroorganismus, ausgewählt aus *Porphyromonas sp., vorzugsweise Porphyromonas gingivalis, Treponema sp., vorzugsweise Treponema denticola,* oder *Actinobacillus sp., vorzugsweise Actinobacillus actinomycetemcomitans,* zur Ausbildung eines Biofilmes, b) Zugabe des zu untersuchenden Mikroorganismus der Gattung *Lactobacillus* und Inkubation des Ansatzes zur Ausbildung der spezifischen Bindung zwischen dem oralpathogenen Mikroorganismus und dem zu untersuchendem Mikroorganismus der Gattung *Lactobacillus* c) Abtrennen der nicht gebundenen Mikroorganismen der Gattung *Lactobacillus,* d) Bestimmung des Biofilmes im Hinblick auf gebundene und aggregierte Mikroorganismen der Gattung *Lactobacillus,* und e) Untersuchen der Hemmung der Bildung eines Biofilms durch den zumindest einen oralpathogenen Mikroorganismus

Das Abtrennen in Schritt c) kann dabei bspw. und vorzugsweise mittels Zentrifugieren zur Bildung eines Überstands oder durch Abkippen vorgenommen werden.

Insbesondere ist dabei bevorzugt, wenn die zu untersuchenden Mikroorganismen im Ansatz markiert sind, vorzugsweise Fluoreszenz-markiert, und dass der durch die oralpathogenen Mikroorganismen ausgebildete Biofilm im Hinblick auf eine Zunahme der Fluoreszenz in Folge einer Bindung der Mikroorganismen der Gattung Lactobacillus an den Biofilm bestimmt wird. Dieses Verfahren bietet den Vorteil, dass die erfindungsgemäßen Mikroorganismen gezielt dahingehend untersucht und identifiziert werden können, ob sie zur spezifischen Bindung der erwähnten oralpathogenen Mikroorganismen geeignet sind. Wenn die zu testenden Mikroorganismen die oralpathogenen Mikroorganismen nicht binden, so können diese planktonischen Zellen bspw. durch eine höhere Fluoreszenz im Überstand bestimmt werden.

Erfindungsgemäß weist das Verfahren den Schritt der Untersuchung der Biofilm-Hemmung durch die oralpathogenen Mikroorganismen auf. Hierbei werden bevorzugt Fluoreszenz-markierte oralpathogene Mikroorganismen eingesetzt. Die Zugabe der zu untersuchenden Mikroorganismen der Gattung Lactobacillus erfolgt dabei während der Inkubation der Biofilm-bildenden oralpathogenen Mikroorganismen. Nach Entfernung der nicht gebundenen Zellen erfolgt eine Quantifizierung der Biofilmbildung durch Messung der Fluoreszenz der im Biofilm gebundenen und Fluoreszenz-markierten oralpathogenen Mikroorganismen im Vergleich zu einer Kontrolle ohne Zugabe der zu testenden Mikroorganismen.

Es versteht sich, dass die vorstehend genannten und nachstehend noch zu erläuternden Merkmale nicht nur in der jeweils angegebenen Kombination, sondern auch in Alleinstellung verwendbar sind, ohne den Rahmen der vorliegenden Erfindung zu verlassen.

### KURZE BESCHREIBUNG DER FIGUREN

Weitere Vorteile ergeben sich aus den nachstehenden Figuren und den hierzu durchgeführten Versuchen und Ausführungsbeispielen. Es zeigen:

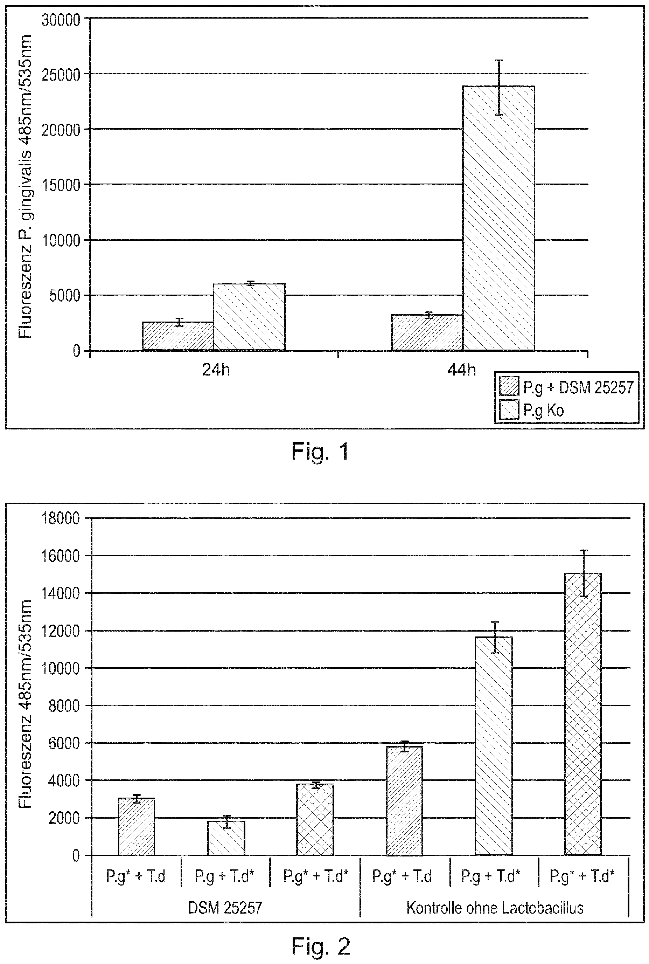

- Fig. 1: den Einfluss eines Ausführungsbeispiels eines erfindungsgemäßen Mikroorganismus (DSM 25257) auf die Biofilmausbildung von *Porphyromonas gingivalis;* spezifische Quantifizierung der Bindung (8-fach Bestimmung) von CFDA-markierten *Porphyromonas gingivalis* an die 96-Well-Mikortiterplatte über Fluoreszenzmessung (485 nm/535 nm);
- Fig. 2: die Inhibierung der Biofilmbildung anhand eines Ausführungsbeispiels eines erfindungsgemäßen Mikroorganismus (DSM 25257) von *Porphyromonas gingivalis* und *Treponema denticola* mittels *in vitro* statischem dualen Species Biofilm Modell; spezifische Quantifizierung der Bindung über Fluoreszenzmessung der gebundenen, mit CFDA-markierten Targetkeime, 8-fach Bestimmung im 9-Well-format;

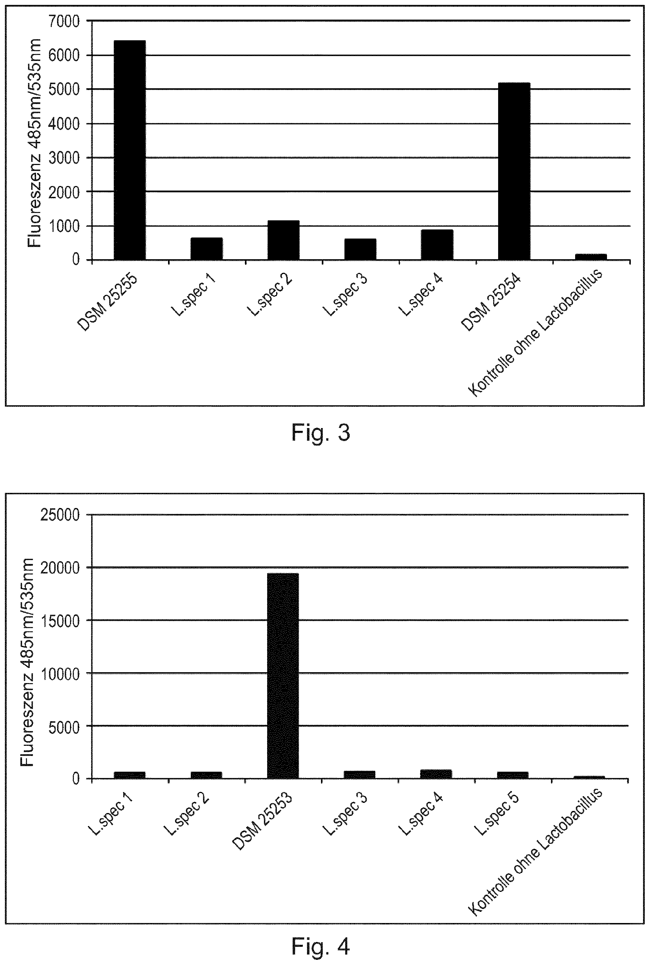

- Fig. 3: die spezifische Bindung von Ausführungsbeispielen eines erfindungsgemäßen Mikroorganismus (DSM 25255 und DSM 25254) an einen durch *Actinobacillus actinomycetemcomitans* ausgebildeten Biofilm (Bindungs-Assay) sowie im Vergleich dazu weitere nicht erfindungsgemäße Lactobacillus Stämme, die nicht zur Bindung an *Actinobacillus actinomycetemcomitans* befähigt sind (*L. spec*); spezifische Quantifizierung der Bindung (8-fach Bestimmung) von CFDA-markierten erfindungsgemäßem *Lactobacilli* an den Biofilm in einer 96-Well-Mikrotiterplatte über Fluoreszenzmessung (485/535nm);
- Fig. 4: die spezifische Bindung eines Ausführungsbeispiels eines erfindungsgemäßen Mikroorganismus (DSM 25253) an einen durch *Treponema denticola* ausgebildeten Biofilm (Bindungs-Assay) sowie im Vergleich dazu weitere, nicht erfindungsgemäße *Lactobacillus* Stämme, die nicht zur Bindung an *Treponema denticola* befähigt sind (*L. spec*); spezifische Quantifizierung der Bindung (8-fach Bestimmung) von CFDA-markierten erfindungsgemäßem Lactobacillus an den Biofilm in 96-Well-Mikrotiterplatte über Fluoreszenzmessung (485/535nm).

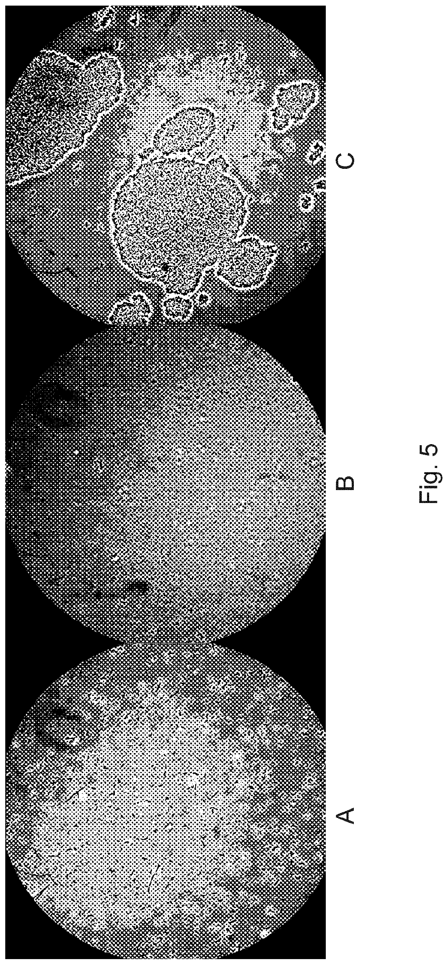

- Fig. 5: die mikroskopische Kontrolle der spezifischen Bindung eines Ausführungsbeispiels eines erfindungsgemäßen Mikroorganismus (DSM 25253) an *Porphyromonas gingivalis* Zellen; Mikroskopische Abbildung (Phasenkontrast, 1000x vergrößert).

### DETAILLIERTE BESCHREIBUNG DER AUSFÜHRUNGSBESPIELE

Zur Identifizierung und Selektion von erfindungsgemäßen Mikroorganismen wurden aus einer *Lactobacillus*-Datenbank verschiedene Stämme durch ein vier-stufiges Screening untersucht, wobei zunächst hinsichtlich der Bindungsfähigkeit an orale pathogene Bakterien (im Folgenden auch "Targetkeime" oder "Targetstamm") gescreent wurde (Bindungs-Assay), und anschließend die im ersten Schritt identifizierten Stämme in einem Co-Aggregationsassay im Mikrotiterplatten-Maßstab geprüft wurden, wobei die Co-Aggregation mit dem jeweiligen Targetkeim qualitativ mittels binokularem Stereomikroskop nachgewiesen wurde. Ferner wurde die Stärke der Co-Aggregation und die Stabilität der Bindung mit dem Targetkeim, sowie die Fähigkeit zur Biofilmverhinderung untersucht, was schließlich zu den identifizierten beispielhaften erfindungsgemäßen Mikroorganismen (*Lactobacillus*) führte.

### Bindungs-Assay

Um die Bindungsaktivität selektierter *Lactobacillus* Stämme quantifizieren zu können, wurde ein Bindungs-Assay etabliert, der den quantitativen Nachweis der Bindung der *Lactobacillus* Stämme an oralpathogene Mikroorganismen in einer 96-Well-Platte ermöglichte. Die Bindungsaktivität der *Lactobacillus* Zellen mit den oralpathogenen Keimen korreliert mit der Co-Aggregationsaktivität bzw. Co-Aggregationsfähigkeit. Dies wurde experimentell überprüft. Dazu zeigt Abbildung 5 exemplarisch die Co-Aggregation des Lactobacillus DSM 25253 und *Porphyromonas gingivalis* (A: *P. gingivalis;* B: Lactobacillus DSM 25253; C: *P. gingivalis* und Lactobacillus DSM 25253).

Die Messmethode basiert auf der spezifischen Bindung der erfindungsgemäßen Stämme an in einem Biofilm gebundene Targetstämme. Für den Assay werden definierte Mengen der erfindungsgemäßen Stämme mit einem Fluoreszenzfarbstoff (CFDA-SE, Invitrogen) markiert und mit einer definierten Menge an in einem Biofilm gebundenen Targetkeimen gemischt; alternativ wird die definierte Menge an erfindungsgemäßem und markiertem Stamm zu einem durch Targetstämme ausgebildeten Biofilm gegeben.

Zur Durchführung dieser Versuche wurden beispielhaft die Targetkeime *Porphyromonas gingivalis* (DSM 20709), *Treponema denticola* (DSM 14222) und *Actinobacillus actinomycetemcomitans* (DSM 11132) nach Standardprotokollen kultiviert. Für *Porphyromonas gingivalis* und *Actinobacillus actinomycetemcomitans* wurde jeweils anaerobes BHI (Brain Heart Infusion)-Medium mit NaHCO₃, supplementiert mit Heminchlorid und Vitamin K1, eingesetzt; für *Treponema denticola* wurde anaerobes DSMZ Medium 909 (DSMZ, Braunschweig) eingesetzt.

Die *Lactobacillus-*Stämme wurden in MRS-Medium (siehe de Man et al., (1960) "A medium for the Cultivation of Lactobacilli", J. Appl. Bact. 23(130-135) anaerob bei 37 °C kultiviert.

Zur Aufarbeitung der Targetkeime wurden die Zellen jeweils nach Erreichen der mittleren exponentiellen Wachstumsphase geerntet, 2 Mal mit Phosphat-gepufferter Saline (PBS, pH 7,4) gewaschen und in PBS aufgenommen. Dann wurden 100 µl der Suspension pro Well in eine 96-Well-Mikrotiterplatte vorlegt.

Während einer 20stündigen anaeroben Inkubation kommt es zur Ausbildung eines Biofilmes durch die Targetstämme. Nach der Inkubation werden nicht gebundene Zellen durch 2-maliges Waschen mit Phosphat-gepufferter Saline (PBS, pH 7,4) entfernt.

Zur Aufarbeitung der *Lactobacillus-*Stämme wurden diese nach 24stündiger Kultivierung geerntet, 2 Mal mit PBS gewaschen, in PBS aufgenommen und durch Zugabe der CFDA-SE-Lösung (Invitrogen) fluoreszenzmarkiert.

Zur Durchführung des Bindungs-Assays wurden zu den in einem Biofilm gebundenen Targetstämmen pro Well 100µl der *Lactobacillus*-Suspension hinzugefügt. Parallel erfolgten Kontrollansätze ohne Zugabe der Lactobacillen-Suspension. Nach 1-stündiger Inkubation bei 30 °C in einem Brutschrank wurden die nicht gebundenen Zellen abgetrennt. Anschließend wurde die Fluoreszenz im Fluoreszenzplattenphotometer (Em485nm/Ex535nm) gemessen. Zusätzlich wurden die Co-Aggregate aus gebundenem Targetstamm und *Lactobacillus* mikroskopisch (Phasenkontrast, 1000x, Öl, Fluoreszenzfilter) bewertet. Dazu zeigt Fig. 5 exemplarisch die Co-Aggregation des *Lactobacillus* DSM 25253 und *Porphyromonas gingivalis* (A: *P. gingivalis;* B: Lactobacillus DSM 25253; C: *P. gingivalis* und Lactobacillus DSM 25253).

Die Zunahme der Fluoreszenz (Em 485 nm/Ex 535 nm) im Ansatz mit *Lactobacillus* Stämmen im Vergleich zur Kontrolle ohne *Lactobacillus* Zellen korrelierte dabei mit der Menge an Biofilm-gebundenen *Lactobacillus* Zellen, Die gemessene Fluoreszenz nach Bindung der *Lactobacillus* Zellen entsprach der Bindungsstärke. Je größer dieser Wert war, desto besser banden die markierten *Lactobacillus* Zellen an die im Biofilm gebundenen Targetstämme, und desto stärker ist die Bindungsaktivität der untersuchten *Lactobacillus* Zellen.

Die Versuche ergaben, dass die selektierten *Lactobacillus* Stämme für die Targetkeime/Targetstämme *Porphyromonas gingivalis* und/oder *Treponema denticola* und/oder *Actinobacillus actinomycetemcomitans* im Bindungs-Assay nach Entfernung der ungebundenen Zellen zu einer 40-95%igen Erhöhung der Fluoreszenz infolge der Bindung der markierten Lactobacillus Zellen an die im Biofilm gebundenen Targetstämme führte. Beispielhaft für die erfindungsgemäßen Mikroorganismen sind in Figur 3 die Ergebnisse des Bindungs-Assays für Lactobacillus DSM 25254 und DSM 25255 mit *Actinobacillus actinomycetemcomitans,* sowie in Figur 4 die Ergebnisse des Bindungs-Assays für Lactobacillus DSM 25253 mit *Treponema denticola* dargestellt. Weiter sind in den beiden Figuren 3 und 4 zum Vergleich die Daten nicht erfindungsgemäßer *Lactobacillus* Stämme dargestellt (*L. spec*), die nicht zur spezifischen Bindung an die Targetkeime befähigt sind.

### Biofilmhemmung

Zur Untersuchung ihrer Wirkung auf die Biofilmbildung der pathogenen Targetkeime *Porphyromonas gingivalis, Treponema denticola* und *Actinobacillus actinomycetemcomitans* wurden die Lactobacillen-Stämme direkt zu dem Fluoreszenz-markierten Targetkeim zu Beginn der Biofilmausbildung zugegeben und für bis zu 48 h inkubiert. Nach der Entfernung der nicht gebundenen Zellen und 2 x Waschen der Biofilme mit PBS erfolgte die Quantifizierung der Biofilme durch Fluoreszenzmessung des gebundenen Targetkeims.

Zur Durchführung dieser Versuche wurden die Targetstämme, wie weiter oben beschrieben, nach Standard-Protokoll kultiviert, sowie die *Lactobacillus-*Stämme in MRS-Medium anaerob kultiviert. Zur Aufarbeitung der Targetstämme wurden diese bis zum Erreichen der mittleren exponentiellen Wachstumsphase kultiviert, geentet und auf die OD600ₙₘ₍ₘₗ₋₁₎=6,0 eingestellt, 2 Mal mit PBS gewaschen und in PBS aufgenommen. Anschließend wurde die CFDA-SE Lösung (Invitrogen, siehe oben; 10 µM Endkonzentration), hinzugegeben, zwei Mal mit PBS gewaschen und das Pellet in PBS aufgenommen.

Zur Aufarbeitung der *Lactobacillus*-Stämme wurden diese kultiviert und auf die OD600ₙₘ₍ₘₗ₋₁₎=12,0 mit PBS eingestellt und, ebenfalls 2 Mal mit PBS gewaschen. Nach der zweiten Waschung wurden die Zellen in PBS-Volumen aufgenommen, welches der OD600ₙₘ₍ₘₗ₋₁₎=12,0 entspricht.

Zur Durchführung des Biofilmbildungs-Assays wurden die *Lactobacillus* Stämme direkt zu dem Fluoreszenz-markierten Targetkeim zu Beginn der Biofilm-ausbildung zugegeben und für 24 h bzw. länger, anaerob, bei 37 °C inkubiert. Parallel erfolgten Kontrollansätze ohne Zugabe der Lactobacillen-Suspension. Nach der Entfernung der planktonischen Zellen und Waschen der Biofilme erfolgte die Quantifizierung der Biofilme durch Fluoreszenzmessung des gebundenen Targetkeimes. Hierzu wurden die Platten mit den trockenen Biofilmen im Fluoreszenzplattenfotometer (485 nm/535 nm) vermessen. Die Reduktion in der Zunahme der Fluoreszenz im Vergleich zur Kontrolle ohne Lactobacillen korrelierte dabei mit der Stärke der Biofilmverhinderung. Beispielhaft für die erfindungsgemäßen Mikroorganismen ist in Figur 1 das Ergebnis der Biofilmverhinderung von *Porphyromonas gingivalis* durch Lactobacillus DSM 25257 dargestellt.

Ferner wurden Versuche durchgeführt, um den Einfluss der Lactobacillen auf die duale Biofilmausbildung von gleichzeitig *Porphyromonas gingivalis* und *Treponema denticola* zu untersuchen. Hierzu wurde beispielhaft der *Lactobacillus* Stamm DSM 25257, der sich als bester Hemmer der Biofilmbildung von *P. gingivalis* erwiesen hat, ausgewählt. Der *Lactobacillus* Stamm wurde zu Beginn der Inkubation zu den zwei pathogenen Targetkeimen zugesetzt und die Verhinderung der Biofilm-bildung von *P. gingivalis* und *T. denticola* nach 22 h untersucht. Es wurden drei Ansätze durchgeführt (siehe Fig. 2). Im ersten Ansatz wurde nur ein Targetkeim (P. *gingivalis*) mit CFDA markiert, in dem zweiten Ansatz wurde der andere Targetkeim (*T. denticola*) markiert und im dritten Ansatz wurden beide Targetkeime mit Fluoreszenz markiert. Dadurch war es möglich, die Zellmenge sowohl der einzelnen Targetkeime im Biofilm als auch die Zellmenge beider Targetkeime zu quantifizieren.

Die Ergebnisse sind in dem in Figur 2 dargestellten Diagramm gezeigt, in welchem die Fluoreszenz-markierten Targetstämme mit einem Sternchen (*) gekennzeichnet sind. Die jeweils linken Balken für die jeweils drei unterschiedlichen Ansätze zeigen die Fluoreszenz des gebundenen *Porphyromonas gingivalis,* die jeweils mittleren Balken die Fluoreszenz vom gebundenen *Treponema denticola* und die jeweils rechten Balken die Bindung beider Targetkeime. Der Ansatz ganz rechts ist die Kontrolle jeweils ohne Zusatz der Lactobacillen.

In diesen Versuchen zeigte sich, dass der *Lactobacillus* Stamm DSM 25257 in der Lage ist, die Biofilmbildung sowohl einzelner Targetkeime als auch die Ausbildung eines dualen Biofilmes bis zu über 50 % zu reduzieren. Auch die Langzeitwirkung (über 168 h) des Stammes gegen einen *Porphyromonas gingivalis*-Biofilm wurde geprüft. Die Hemmung war über den gemessenen Zeitraum stabil. Außerdem wurde gezeigt, dass nach dem Behandeln der Zellen über 30 min bei 70 °C die Hemmaktivität unverändert blieb (Daten nicht gezeigt).

Des Weiteren wurden weitere Untersuchungen der selektierten *Lactobacillus-*Stämme hinsichtlich des Temperatur-Verhaltens und Nebenwirkungen auf einen kommensalen Mundkeim (*Streptococcus salivarius*) im Bindungs-Assay durchgeführt. Hierbei zeigte sich, dass für keinen der erfindungsgemäßen Stämme eine Bindung an *Streptococcus salivarius* nachgewiesen werden konnte (Daten nicht gezeigt).

## Patentansprüche

1. Mikroorganismus, der zur Gattung der Milchsäurebakterien gehört, oder Fragment davon, wobei der Mikroorganismus oder das Fragment davon an zumindest einen oralpathogenen Mikroorganismus spezifisch binden kann, und wobei der zumindest eine oralpathogene Mikroorganismus ausgewählt ist aus der Gruppe *Porphyromonas sp., Treponema sp. oder Actinobacillus sp,* **dadurch gekennzeichnet, dass** der Mikroorganismus die Fähigkeit zur Hemmung der Bildung eines Biofilms des zumindest einen oralpathogenen Mikroorganismus besitzt, und dass der Mikroorganismus ausgewählt ist aus einem Mikroorganismus mit der Hinterlegungsnummer DSM 25257, der Hinterlegungsnummer DSM 25253, der Hinterlegungsnummer DSM 25254 und der Hinterlegungsnummer DSM 25255, oder Fragmente davon, welche die Fähigkeit zur Hemmung der Bildung eines Biofilms des zumindest einen oralpathogenen Mikroorganismus besitzen.

2. Mikroorganismus oder Fragment davon nach Anspruch 1, **dadurch gekennzeichnet, dass** die Fähigkeit zur spezifischen Bindung an den zumindest einen oralpathogenen Mikroorganismus auch in Gegenwart von natürlichem Speichel besteht.

3. Mikroorganismus oder Fragment davon nach einem der Ansprüche 1 oder 2, **dadurch gekennzeichnet, dass** die Fähigkeit zur spezifischen Bindung an den zumindest einen oralpathogenen Mikroorganismus bei einem pH-Wert von zwischen ca. 2,5 bis ca. 9 besteht.

4. Mikroorganismus nach einem der Ansprüche 1 bis 3, **dadurch gekennzeichnet, dass** er über keine Fähigkeit zur Bindung an *Streptococcus salivarius* verfügt.

5. Mikroorganismus nach einem der Ansprüche 1 bis 4, **dadurch gekennzeichnet, dass** die Fähigkeit zur spezifischen Bindung an den zumindest einen oralpathogenen Mikroorganismus auch nach einer Hitzebehandlung bei mindestens ca. 70 °C für mindestens ca. 20 min besteht

6. Mikroorganismus oder Fragment davon nach einem der Ansprüche 1 bis 5, **dadurch gekennzeichnet, dass** er nicht an *Streptococcus salivarius* bindet oder co-aggregiert und dass er zumindest eines der folgenden Merkmale aufweist:
a) Hitzeresistenz,
b) Fähigkeit zur spezifischen Bindung von zumindest einem oralpathogenen Mikroorganismus, und/oder
c) Fähigkeit zur Hemmung der Biofilmbildung durch den zumindest einen oralpathogenen Mikroorganismus.

7. Zusammensetzung, umfassend zumindest einen Mikroorganismus gemäß einem der Ansprüche 1 bis 6, oder Fragment davon, welches die in den Ansprüchen 1 bis 6 definierten Eigenschaften des erfindungsgemäßen Mikroorganismus besitzt, sowie zumindest einen Träger oder Exzipienten.

8. Zusammensetzung nach Anspruch 7, zur Verwendung in der Dentalhygiene, - therapie und -prophylaxe von Säugetieren.

9. Zusammensetzung nach Anspruch 7 oder 8, zur Verwendung in der Therapie und/oder Prophylaxe von Parodontitis, Periimplantitis und Gingivitis in der Human- oder Veterinärmedizin.

10. Zusammensetzung nach einem der Ansprüche 7 bis 9, **dadurch gekennzeichnet, dass** die Zusammensetzung als Nahrungsmittel, Nahrungsmittelzusatz, Hygieneprodukt, Futtermittel, Futtermittelzusatz oder als eine pharmazeutischen Präparation eingesetzt wird.

11. Mikroorganismus nach einem der Ansprüche 1 bis 6, oder Fragment davon, welches die in den Ansprüchen 1 bis 6 definierten Eigenschaften des erfindungsgemäßen Mikroorganismus noch besitzt, zur Verwendung in der Therapie oder Prophylaxe von Parodontitis, Perümplantitis und/oder Gingivitis.

12. Verfahren zur Identifizierung und/oder Selektion eines Mikroorganismus, der zur Gattung der Milchsäurebakterien gehört, wobei der Mikroorganismus an zumindest einen oralpathogenen Mikroorganismus spezifisch binden kann, und wobei der zumindest eine oralpathogene Mikroorganismus ausgewählt ist aus der Gruppe *Porphyromonas sp., Treponema sp. oder Actinobacillus sp,* **dadurch gekennzeichnet, dass** es zumindest die folgenden Schritte aufweist:
a) Inkubieren eines Ansatzes aus einem oralpathogenen Mikroorganismus, ausgewählt aus *Porphyromonas sp., Treponema sp.* oder *Actinobacillus sp.,* zur Ausbildung eines Biofilmes,
b) Zugabe des zu untersuchenden Mikroorganismus der Gattung *Lactobacillus* und Inkubation des Ansatzes zur Ausbildung der spezifischen Bindung zwischen dem oralpathogenen Mikroorganismus und dem zu untersuchendem Mikroorganismus der Gattung *Lactobacillus,*
c) Abtrennen der nicht gebundenen Mikroorganismen der Gattung *Lactobacillus;*
*d)* Bestimmung des Biofilmes im Hinblick auf gebundene und aggregierte Mikroorganismen der Gattung *Lactobacillus; und*
e) Untersuchen der Hemmung der Bildung eines Biofilms durch den zumindest einen oralpathogenen Mikroorganismus.

13. Verfahren nach Anspruch 12, **dadurch gekennzeichnet, dass** die oralpathogenen Mikroorganismen oder der zu untersuchende Mikroorganismus der Gattung *Lactobacillus* mit einem Fluoreszenz-Marker markiert sind.

14. Verfahren nach Anspruch 12 oder 13, **dadurch gekennzeichnet, dass** die Untersuchung der Hemmung der Biofilm-Ausbildung die folgenden Schritte aufweist:
i. Inkubieren von Mikroorganismen der Gattung *Lactobacillus sp.* mit dem zumindest einen oralpathogenen Mikroorganismus, zur Beginn der Biofilmausbildung durch den zumindest einen oralpathogenen Mikroorganismus,
ii. Quantifizierung der Biofilme.

## Claims

1. Microorganism belonging to the genus of lactic acid bacteria or fragment thereof, wherein the microorganism or fragment thereof is capable of specifically binding to at least one oral pathogenic microorganism, and wherein the at least one oral pathogenic microorganism is selected from the group comprising *Porphyromonas sp., Treponema sp. or Actinobacillus sp,* **characterized in that** the microorganism has the capability of inhibiting the formation of a biofilm of the at least one oral pathogenic microorganism, and that the microorganism is selected from a microorganism having the accession number DSM 25257, the accession number DSM 25253, the accession number DSM 25254, and the accession number DSM 25255, or fragments thereof, which have the capability of inhibiting the formation of a biofilm of the at least one oral pathogenic microorganism.

2. The microorganism or fragment thereof according to claim 1, **characterized in that** the capability of specifically binding to the at least one oral pathogenic microorganism is also persisting in presence of natural saliva.

3. The microorganism or fragment thereof according to claim 1 or 2, **characterized in that** the capability of specifically binding to the at least one oral pathogenic microorganism is persisting at a pH value of between about 2,5 to about 9.

4. The microorganism according to any of claims 1 to 3, **characterized in that** it is not capable of binding to *Streptococcus salivarius.*

5. The microorganism or fragment thereof according to any of claims 1 to 4, **characterized in that** the capability of specifically binding to the at least one oral pathogenic microorganism is persisting even after heat treatment at at least 70°C for at least 20 min.

6. The microorganism or fragment thereof according to any of claims 1 to 5, **characterized in that** it does not bind to or co-aggregate with *Streptococcus salivarius* and that it comprises at least one of the following characteristics:
a) heat resistance,
b) the capability of specifically binding to at least one oral pathogenic microorganism, and/or
c) the capability of inhibiting of the formation of a biofilm through the at least one oral pathogenic microorganism.

7. Composition, comprising at least one microorganism according to any of claims 1 to 6, or a fragment thereof, which has the characteristics of the microorganism according to the invention as claimed in any of claims 1 to 6, and comprising at least one carrier or excipient.

8. The composition according to claim 7, for use in dental hygiene, dental therapy and dental prophylaxis of mammals.

9. The composition according to claim 7 or 8, for use in the therapy and/or prophylaxis of periodontitis, peri-implantitis and gingivitis in human and veterinary medicine.

10. The composition according to any of claims 7 to 9, **characterized in that** the composition is used as foodstuff, food supplement, sanitary product, animal feed, animal feed supplement, or as a pharmaceutical preparation.

11. The microorganism according to any of claims 1 to 6, or fragment thereof, which still has the characteristics of the microorganism according to the invention as defined in claims 1 to 6, for use in therapy and/or prophylaxis of periodontitis, periimplantitis and gingivitis.

12. Method for identifying and/or selecting a microorganism, which belongs to the genus of lactic acid bacteria, wherein the microorganism is capable of specifically binding to at least one oral pathogenic microorganism, and wherein the at least one oral pathogenic microorganism is selected from the group comprising *Porphyromonas sp., Treponema sp. or Actinobacillus sp,* **characterized in that** it comprises at least the following steps:
a) incubating a batch comprising an oral pathogenic microorganism selected from *Porphyromonas sp., Treponema sp.* or *Actinobacillus sp.,* in order to form a biofilm,
b) adding the microorganism of the genus *Lactobacillus* to be assayed and incubating the batch in order to form a specific binding between the oral pathogenic microorganism and the microorganism of the genus *Lactobacillus* to be tested,
c) separating the unbound microorganism of the genus *Lactobacillus;*
d) determining of the biofilm with respect of bound and aggregated microorganism of the genus *Lactobacillus;* and
e) assessing the inhibition of the formation of the biofilm through the at least one oral pathogenic microorganism.

13. The method of claim 12, **characterized in that** the oral pathogenic microorganisms or the microorganism of the genus *Lactobacillus* to be assayed are labeled with a fluorescence marker.

14. The method of claim 12 or 13, **characterized in that** the assessment of the inhibition of the formation of the biofilm comprises the following steps:
i. incubating of microorganisms of the genus *Lactobacillus sp.* with the at least one oral pathogenic microorganism at the outset of the formation of the biofilm through the at least one oral pathogenic microorganism,
ii. quantifying of the biofilms.

## Revendications

1. Micro-organisme, qui appartient au genre des bactéries lactiques, ou fragment de celui-ci, le micro-organisme ou le fragment de celui-ci pouvant se fixer spécifiquement à au moins un micro-organisme pathogène de la cavité buccale, et ledit au moins un micro-organisme pathogène de la cavité buccale étant choisi dans le groupe constitué par *Porphyromonas sp., Treponema sp.* et *Actinobacillus sp.,* **caractérisé en ce que** le micro-organisme possède l'aptitude à inhiber la formation d'un biofilm dudit au moins un micro-organisme pathogène de la cavité buccale, et **en ce que** le micro-organisme est choisi parmi les micro-organismes portant le numéro de dépôt DSM 25257, le numéro de dépôt DSM 25253, le numéro de dépôt DSM 25254 et le numéro de dépôt DSM 25255, ou des fragments de ceux-ci, qui possèdent l'aptitude à inhiber la formation d'un biofilm dudit au moins un micro-organisme pathogène de la cavité buccale.

2. Micro-organisme ou fragment de celui-ci selon la revendication 1, **caractérisé en ce que** l'aptitude à la fixation spécifique audit au moins un micro-organisme pathogène de la cavité buccale existe également en présence de salive naturelle.

3. Micro-organisme ou fragment de celui-ci selon l'une quelconque des revendications 1 et 2, **caractérisé en ce que** l'aptitude à la fixation spécifique audit au moins un micro-organisme pathogène de la cavité buccale existe à un pH compris entre environ 2,5 et environ 9.

4. Micro-organisme selon l'une quelconque des revendications 1 à 3, **caractérisé en ce qu'**il n'est doté d'aucune aptitude à la fixation à *Streptococcus salivarius.*

5. Micro-organisme selon l'une quelconque des revendications 1 à 4, **caractérisé en ce que** l'aptitude à la fixation spécifique audit au moins un micro-organisme pathogène de la cavité buccale existe également après un traitement par la chaleur à au moins environ 70 °C pendant au moins environ 20 minutes.

6. Micro-organisme ou fragment de celui-ci selon l'une quelconque des revendications 1 à 5, **caractérisé en ce qu'**il ne se fixe pas à ni ne co-agrège avec *Streptococcus salivarius* et **en ce qu'**il présente au moins l'une des caractéristiques suivantes :
a) résistance à la chaleur,
b) aptitude à la fixation spécifique d'au moins un micro-organisme pathogène de la cavité buccale, et/ou
c) aptitude à l'inhibition de la formation de biofilm par ledit au moins in micro-organisme pathogène de la cavité buccale.

7. Composition, comprenant au moins un micro-organisme selon l'une quelconque des revendications 1 à 6, ou un fragment de celui-ci, qui possède les propriétés du micro-organisme selon l'invention, définies dans les revendications 1 à 6, ainsi qu'au moins un véhicule ou des excipients.

8. Composition selon la revendication 7, destinée à l'utilisation dans l'hygiène, la thérapie et la prophylaxie dentaires de mammifères.

9. Composition selon la revendication 7 ou 8, destinée à l'utilisation dans la thérapie et/ou la prophylaxie de la parodontite, la péri-implantite et la gingivite en médecine humaine ou vétérinaire.

10. Composition selon l'une quelconque des revendications 7 à 9, **caractérisée en ce que** la composition est utilisée comme produit alimentaire, additif à un produit alimentaire, produit d'hygiène, aliment pour animaux, additif à un aliment pour animaux ou sous forme d'une préparation pharmaceutique.

11. Micro-organisme selon l'une quelconque des revendications 1 à 6 ou fragment de celui-ci, qui possède les propriétés du micro-organisme selon l'invention, définies dans les revendications 1 à 6, destiné à l'utilisation dans la thérapie ou la prophylaxie de la parodontite, la péri-implantite et/ou la gingivite.

12. Procédé pour l'identification et/ou la sélection d'un micro-organisme qui appartient au genre des bactéries lactiques, le micro-organisme pouvant se fixer spécifiquement à au moins un micro-organisme pathogène de la cavité buccale et ledit au moins un micro-organisme pathogène de la cavité buccale étant choisi dans le groupe constitué par *Porphyromonas sp., Treponema sp.* et *Actinobacillus sp.,* **caractérisé en ce que** lequel procédé comprend au moins les étapes suivantes :
a) incubation d'une charge initiale à base d'un micro-organisme pathogène de la cavité buccale, choisi parmi *Porphyromonas sp., Treponema sp.* et *Actinobacillus sp.,* pour la formation d'un biofilm,
b) addition du micro-organisme à étudier, appartenant au genre *Lactobacillus,* et incubation de la charge initiale pour la formation de la liaison spécifique entre le micro-organisme pathogène de la cavité buccale et le micro-organisme à étudier, appartenant au genre *Lactobacillus,*
c) séparation des micro-organismes appartenant au genre *Lactobacillus,* non fixés ;
d) détermination du biofilm en ce qui concerne les micro-organismes appartenant au genre *Lactobacillus,* fixés et agrégés ; et
e) étude de l'inhibition de la formation d'un biofilm par ledit au moins un micro-organisme pathogène de la cavité buccale.

13. Procédé selon la revendication 12 ou 13, **caractérisé en ce que** les micro-organismes pathogènes de la cavité buccale ou le micro-organisme à étudier, appartenant au genre *Lactobacillus,* sont marqués avec un marqueur fluorescent.

14. Procédé selon la revendication 12 ou 13, **caractérisé en ce que** l'étude de l'inhibition de la formation de biofilm comporte les étapes suivantes :
i. incubation de micro-organismes appartenant au genre *Lactobacillus sp.* avec ledit au moins un micro-organisme pathogène de la cavité buccale, au début de la formation de biofilm par ledit au moins un micro-organisme pathogène de la cavité buccale,
ii. détermination quantitative des biofilms.
